# EUROPEAN PATENT SPECIFICATION

(11) **EP 3 193 824 B1**
(45) Date of publication and mention of the grant of the patent: **05.07.2023**
(21) Application number: 15836677.3
(22) Date of filing: 26.08.2015
(51) Int. Cl.: A61K 31/198, A61K 31/28, A61K 45/06, A61P 43/00, A61K 33/04, A61K 31/366, A61K 31/40

(54) **A COMPOSITION FOR USE IN TREATING HYPERLIPIDEMIA, HYPERCHOLESTEROLEMIA AND/OR HYPERTRIGLYCERIDEMIA VIA PHYSIOLOGICALLY SYNTHESISED GLUTATHIONE**
ZUSAMMENSETZUNG ZUR VERWENDUNG BEI DER BEHANDLUNG VON HYPERLIPIDEMIE, HYPERCHOLESTEROLEMIE UND/ODER HYPERTRIGLYCERIDEMIE,ÜBER PHYSIOLOGISCH SYNTHETISIERTES GLUTATHION
COMPOSITION POUR SON UTILISATION DANS LE TRAITEMENT DE L'HYPERLIPIDEMIE, DE L'HYPERCHOLESTEROLEMIE ET/OU DE L'HYPERTRIGLYCERIDEMIE PAR L'INTERMÉDIAIRE DE GLUTATHION PHYSIOLOGIQUEMENT SYNTHÉTISÉ

(30) Priority: 29.08.2014 US 201462043854 P
(43) Date of publication of application: 26.07.2017
(73) Proprietor: The ProImmune Company, LLC, Rhinebeck, NY 12572-1606 (US)
(72) Inventor: Crum, Albert, Rhinebeck, NY 12572-1606 (US)
(74) Representative: Tollett, Ian
(86) International application number: PCT/US2015/046949
(87) International publication number: WO 2016/033183

(56) References cited:
- WO-A1-2008/052184
- US-A1- 2002 176 900
- US-A1- 2005 271 726
- US-A1- 2005 271 726
- US-A1- 2008 175 925
- US-A1- 2009 104 287
- US-A1- 2011 129 523
- US-A1- 2012 029 082
- US-B1- 6 592 908
- FEDACKO J ET AL: "Abstract: 604 COENZYME Q10 AND SELENIUM SUPPLEMENTATION IN PATIENTS WITH STATIN-ASSOCIATED MYOPATHY", ATHEROSCLEROSIS SUPPLEMENTS, ELSEVIER, AMSTERDAM, NL, vol. 10, no. 2, 1 June 2009 (2009-06-01), page e357, XP026780623, ISSN: 1567-5688, DOI: 10.1016/S1567-5688(09)70353-8 [retrieved on 2009-06-01]
- D. MOSSHAMMER ET AL: "Mechanisms and assessment of statin-related muscular adverse effects", BRITISH JOURNAL OF CLINICAL PHARMACOLOGY, vol. 78, no. 3, 20 February 2014 (2014-02-20), pages 454-466, XP002781070,
- J. FUHRMEISTER ET AL.: "Prooxidative toxicity and selenoprotein suppression by cerivastatin in muscle cells", TOXICOLOGY LETTERS, vol. 215, 2012, pages 219-227, XP002781071,

## Description

### Technical Field

The present invention relates to a composition comprising a selenium source and a glutathione source as defined in the claims, in combination with a statin, for use in treating hyperlipidemia, hypercholesterolemia and/or hypertriglyceridemia.

Further disclosed embodiments relate generally to certain compositions for use, e.g., compositions comprising a glutathione precursor and a selenium source, in the therapy of subjects suffering from diseases associated with hypertriglyceridemia hyperlipemia, and/or hypercholesterolemia. Particular embodiments of the present invention relate to compositions for use in treatment and/or reducing the incidence of adverse side effects of statin therapy comprising administering to a subject in need, the aforementioned compositions. Embodiments of the invention relate to compositions for use in combination therapy with statins. In other embodiments, the present invention relates to the aforementioned compositions, including combinations with additional pharmaceutical agents, or other non-pharmaceutical agents for use in the therapy of subjects that suffer from disorders related to oxidative stress (O.S.) such as hypertriglyceridemia, hypercholesterolemia and/or hyperlipidemia. Furthermore, the invention can benefit those individuals who suffer adverse side effects from taking statins. The beneficial way that the adverse side effects are ameliorated is by providing adjunct anti-hypertriglyceridemia, anti-hyperlipidemia and anti-hypercholesterolemia, therapy that can reduce the total reliance on statins or other antilipidemic compounds as has been documented by University research.

### Background of the Invention

A University research study article has demonstrated a novel and unexpected "new use," i.e., an interrelationship between the immune system and fat metabolism, using the invention. (See Sinha-Hikim I et al, "A novel cystine based antioxidant attenuates oxidative stress and hepatic steatosis in diet-induced obese mice." Experimental and Molecular Pathology. 2011 August: 91(1):419-428.).

How such a novel and innovative title to describe the laboratory observations for F1 could be set forth is based on the novel and innovative biochemical mechanisms of action for the multi-functional dimensions of the invention. Following are some of the major scientific university documentations in the peer-reviewed Journal Experimental and Molecular Pathology, with findings and results beginning on page 6 of the article. In this article, the invention is referred to as "F1."

Page 6 of the above-mentioned article: "F1 prevents HFD-induced weight gain, hepatomegaly, prevents increased serum triglyceride levels and liver pathology"... "The adverse effects of HFD (high fat diet) on body and liver weights, and serum triglyceride levels were fully prevented by dietary supplementation of F1."

Page 7 of the above-mentioned article: "Dietary supplementation of Fl fully prevented such HFD-induced hepatic steatosis ... in ApoE-/- Mice" ... "Treatment with F1 attenuated such HFD-induced increase in intracellular fat accumulation to levels identical to that seen in ApoE-/- fed with ND (normal diet)." ... "alterations in cellular ultrastructure were fully prevented by dietary supplementation of F1... Apoptotic index in liver of mice fed with HFD (high fat diet) was about 9% but was significantly (P<0.5) reduced in mice fed with HFD plus Fl" ... "HFD-induced oxidative stress in obese mice are suppressed by antioxidant treatment" ... in terms of the GSH/GSSG ratio: "Treatment with F1 significantly (P>0.05) prevented this HFD-induced increase in oxidative stress." ... "The increase in 4-HNE expression in mice fed with HFD was prevented by (F1) antioxidant treatment. This was further corroborated by image analysis of changes in the staining intensity (Fig. 2D)." ... "Dietary supplementation of F1 activates Nrf2 but suppresses lipogenesis through inactivation of AMPK."

Page 8 of the above-mentioned article: "F1 prevents activation of JNK and p38MAPK, perturbation of BAX/BCL-2 ratio, and caspase activation in livers in mice fed with HFD." ... "Dietary supplementation of Fl significantly (P<0.05) suppressed HFD-induced up-regulation of BAX" ... "We are also intrigued by the observations that dietary supplementation of Fl is capable of preventing HFD-induced hypertriglyceridemia, inactivation of AMPK and increased expression of lipogenic enzymes such as FAS."

Page 9 of the above-mentioned article: "...Dietary supplementation of Fl is effective in mitigating HFD-induced liver damage caused by oxidative stress and lipid peroxidation." ... "F1 is further capable of activation of Nrf2 and preventing HFD-induced inhibition of AMPK and in the expression of lipogenic enzymes such as FAS. Nrf2 serves as master regulator of a cellular defense system against oxidative stress (Motohashi and Yamamoto, 2004, Nguyen et al. 2009)."

The novel and innovative biochemical mechanisms of action for the multi-functional dimensions of the invention and the greater effectiveness of this invention over the pharmacological standard N-acetylcysteine are be dealt with later. However, in the above-mentioned article, researchers noted: "....in a recent study, we have shown that compared to F1 ...N-acetylcysteine fortified with the same amount of selenomethionine like F1 is less effective in ameliorating spermine-induced reduction in GSH levels and perturbation in cellular morphology (Sinha-Hikim et al. 2010b)."

Statins are routinely used in cancer chemotherapeutics and the treatment of diseases associated with hyperlipidemia and/or hypercholesterolemia. Mechanistically, they act by inhibiting HMG-CoA reductase, the rate-controlling enzyme (EC 1.1.1.88 & EC 1.1.1.34) of the mevalonate pathway, i.e., the metabolic pathway that produces cholesterol and other isoprenoids. Continued and prolonged use of statins is known to give rise to potentially serious side effects. These effects can be disabling and may last for the duration of the causative drug treatment or even after the drug treatment is complete, affecting not only an individual's capacity to work but also perform the simple tasks involved in day to day life.

Later in this document the very words and warnings of the pharmaceutical manufacturers of statins will be set forth to outline the down-side effects of statins. A peer-reviewed article about the likelihood potentially of diabetes following statin treatment is hereby presented: Carter AA, et al in "Risk of incident diabetes among patients treated with statins: population based study", BMJ 2013:346:12610 May 23, 2013). Under "Clinical Evidence" the following statement is made: "Meta analyses also showed that statin therapy was associated with a 9% to 13% increased risk for incident diabetes."

Statins are used in the treatment and management of diseases associated with hypertriglyceridemia, hyperlipidemia and/or hypercholesterolemia, e.g., cardiovascular disease (CVD). CVD is the single most common cause of death, accounting for about one fourth of all deaths in the United States in 2010 (Murphy et al. "Deaths: Final data for 2010," Natl Vital Stat Rep. 2013;61(4)). Cardiovascular disease has many causes and is characterized by complex interactions between the heart, blood vessels, peripheral organs and the tissues. Some types of cardiovascular disease such as coronary heart disease (CHD) or stroke occur acutely and are associated with severe consequences, including death. Medically these are managed with aggressive treatment (drugs and surgery) followed by chronic treatment to prevent recurrence. Other types of cardiovascular disease such as hypertension (high blood pressure) and dyslipidemia progress slowly, and are usually managed by diet and long-term therapy.

The relationship between hypercholesterolemia (excessive blood cholesterol levels) and low density lipoproteins and increased incidence of coronary artery (heart) disease has also been well-established in literature and statins are increasingly used to treat and manage such diseases. Although cholesterol is an essential component of a healthy functioning body, being required for the formation of functional membranes, steroid hormones and bile acids, excessive levels, particularly when associated with low density lipoproteins (LDLs), constitute a health risk. Of the deaths resulting from cardiovascular disease, more than three quarters can be attributed to atherosclerosis and its complications.

Atherosclerosis is a generalized disease of the arteries that often develops in a symptom free manner over many years. The most common outcomes of atherosclerosis are incidences of coronary heart disease, with other outcomes being incidences of stroke and peripheral heart disease. Elevated blood cholesterol concentration and high levels of low density lipoproteins are a major contributing factor in the development of atherosclerosis. In situations of excessive blood cholesterol levels, cholesterol is gradually deposited on the artery walls together with other fats, resulting in plaque build-up which disrupts the free flow of blood, with potentially severe results.

The following blood biomarker levels (normally expressed as mg/dL) are considered adverse or pathological in standard medical practice: cholesterol over 200 mg/dL; low density lipoproteins (LDL) over 130 mg/dL; very low density lipoproteins (vLDL) over 30 mg/dL; and/or triglycerides over 150 mg/dL. See also, Stone et al. "ACC/AHA Guideline on the Treatment of Blood Cholesterol to Reduce Atherosclerotic Cardiovascular Risk in Adults. J Am Coll Cardiol. 2014 Jul 1;63(25 Pt B):2889-934 and American Diabetes Association's "Standards of medical care in diabetes -- 2014." Diabetes Care. 2014;37 Suppl 1:514-580.

To lower high cholesterol levels, patients are treated with a range of drugs, commonly known as the statins, which include atorvastatin (e.g., LIPITOR, TORVAST), cerivastatin (e.g., LIPOBAY, BAYCOL; which were withdrawn from market in August 2001 due to the risk of rhabdomyolysis), fluvastatin (e.g., LESCOL, LESCOL XL), lovastatin (e.g., MEVACOR, ALTOCOR, ALTOPREV), mevastatin (e.g., COMPACTIN), pitavastatin (e.g., LIVALO, PITAVA), pravastatin (e.g., PRAVACHOL, SELEKTINE, LIPOSTAT), rosuvastatin (CRESTOR), simbastatin (e.g., ZOCOR, LIPEX). Combinations of the aforementioned statins (e.g., atorvastatin and lovastatin, etc.) may also be used. These agents act to decrease the level of cholesterol and low density lipoproteins in blood/tissues.

Statin use is not limited to therapy of hypertriglyceridemia, hyperlipidemia and/or hypercholesterolemia, however. Statins have also recently been reported to have potential utility in the treatment of dementia (Jick et al. "Statins and the risk of dementia," The Lancet, 2000: 356; 1627-1631) and various cancers, e.g., prostate, skin, lung colon, bladder, uterus and kidney. See, Boudreau et al. "Statin Use and Cancer Risk: A Comprehensive Review," Expert Opinion Drug Safety, Jul 2010; 9(4): 603-621. Other indications in which statins have been experimented include, for example, treatment of nuclear cataracts and hypertension. See, Klein et al. "Statin use and incident nuclear cataract," JAMA 295 (23): 2752-8 and Golomb et al. "Reduction in blood pressure with statins: results from the UCSD Statin Study, a randomized trial," Arch. Intern. Med. 168 (7): 721-7.

However, there are a number of potentially serious side effects associated with statin therapy, including diabetes, rhabdomyolysis, headache, joint pain, fever, muscle pain, back pain, abdominal cramping, sleep disorder, rhinitis, sinusitis, stimulation of coughing reflex, dizziness and fatigue. Of the contraindications for this group of drugs, two of the most common are fatigue and/or muscle pain (often referred to as "myalgia"). In severe cases, these symptoms may lead to the undesirable cessation of the vital therapy. In rare cases, severe muscle wastage (rhabdomyolysis) has been reported. The risk of adverse side effects during treatment with the statins is increased with concurrent administration of certain other drugs, such as cyclosporin, fibric acid derivatives (e.g., GEMFIBROZIL), erythromycin, niacin or other antifungals. Similar symptoms to those experienced by patients undergoing statin therapy may also be experienced by patients undergoing therapy with other drugs, or may be experienced as a result of a disease state. A university study has found a "10-22% increased risk of diabetes for some statins" was published in 2013 (Carter AA et al, "Risk of incident diabetes among patients treated with statins: population based study." BMJ, 2013:346:12510 doi: 10.1136bmj 12610 May 23, 2013.

In December 2007, a consortium of universities contracted to invest NIH and other research funds -- $25,000,000.00 (million) actually spent-- for research on Patent RE39,734 (RE42,645E). However, as a precondition for such a financial commitment, the Universities had to convince themselves with vigorous investigation on Patent RE39,734 (RE42,645E) so as to determine if Patent RE39,734 (RE42,645E) was more effective than N-acetylcysteine (the pharmaceutical standard for raising bodily intracellular Glutathione levels). The precondition research period demonstrated that Patent RE39,734 (RE42,645E) was superior to N-acetylcysteine (NAC) in raising intracellular glutathione, plus providing other superior immune benefits (See below).

Via detailed comparison research and tests performed, the scientists concluded that Patent RE39,734 (RE42,645E) consistently demonstrated physiological superiority over NAC in the production of glutathione, including the superiority of its various immune protections.

Major university medical centers participated in the research studies that went forward on Patent RE39,734 (RE42,645E). The Universities involved included Charles R. Drew University of Medicine and Research, University of California (Irvine), UCLA (David Geffen School of Medicine), Los Angeles Biomedical Institute, UCLA (Harbor), and the University of Texas Southwestern (Dallas).

Thus, there exists an urgent need for the treatment of and/or reversal of the adverse effects of statin therapy, e.g., muscle pain and fatigue, in patients undertaking statins for one or more of the aforementioned indications.

### Summary of the Invention

Embodiments of the present invention described herein relate to compositions for use in combination with a statin compound in a method for treating hypertriglyceridemia, hypercholesterolemia, and/or hyperlipidemia in a subject in need thereof, wherein the composition comprises a selenium source and a glutathione precursor, which is a mixture of a glutamate source, L-cystine, and glycine. In particular, the adverse effects of statin therapy can be reduced or reversed by administering compositions comprising the glutathione precursor, either simultaneously, sequentially or separately with a selenium source. The compositions described below can therefore provide a useful adjunctive therapy with statins (e.g., allow for a lowered dosage of statins which achieves the same efficacy without the risk of adverse effects) or serve as replacement therapy in subjects who exhibit or are prone to developing adverse side effects.
US2002/176900 A1 discloses compositions for promoting a healthy cardiovascular system and enhancing healthy blood flow in a mammal comprising calcium; magnesium; selenium; manganese; zinc; potassium; vitamin E; vitamin A; alpha-lipoic acid; Allium sativum extract; Medicago sativa extract; Chondrus crispus extract; L-cysteine; L-glutamic acid; glycine; and glutathione.

Embodiments of the present invention relate generally the new and novel use of certain compositions, comprising a glutathione precursor and a selenium (co-factor) source, in the therapy of subjects suffering from diseases associated with hypertriglycerideamia and hyperlipidemia and/or hypercholesterolemia and low density lipoproteins. Related embodiments of the present invention relate to treatment for reducing total reliance on statins and thus reducing the incidence of the adverse side effects related to statin therapy. Related embodiments of the present invention, which comprises the product, and may be administered to a subject to reduce total reliance on statins and incidences of their adverse side effects, relate to the composition comprising a glutathione precursor and a selenium source. Embodiments of the invention also relate to the use of compositions as combination adjunct therapy with other agents such as statins, thus using lower dosages of statins plus cholesterol absorption inhibitors or thus using lower dosages of cholesterol absorption inhibitors, bile acid binding resins or lower dosages of bile acid binding resins, or lower dosages of fibrates. With the benefit of this invention via innate immunity and unique bioavailability of glutathione, it is possible to use less statins. Such compositions comprising the glutathione precursor and the selenium co-factor can also independently reduce hypertriglyceridemia and/or hypercholesterolemia and hyperlipidemia.

Additional embodiments as described herein not falling within the scope of the claimed subject matter relate to uses of the aforementioned compositions comprising the glutathione precursor and the selenium source in the treatment of aging and circulatory conditions.

Additional features and advantages are realized through the techniques of the present invention. Other embodiments and aspects of the invention are described in detail herein and are considered a part of the claimed invention.

The recitation herein of desirable objects which are met by various embodiments of the present invention is not meant to imply or suggest that any or all of these objects are present as essential features, either individually or collectively, in the most general embodiment of the present invention or in any of its more specific embodiments.

### Detailed Description

According to one embodiment of the present disclosure there is described a method of treatment of one or more side effects of statin therapy comprising administering to a subject in need of such treatment an effective amount of a composition comprising glutathione (GSH [reduced form] or GSSG [oxidized form]) precursor and a selenium source. The individual components of the composition are disclosed in detail in Crum et al. (US patent app. pub. No. 2012-0029082). See also US Reissue patent Nos. 39,734 and 42,645. As detailed in Crum et al., the individual components of the compositions include the three amino acids which serve as precursors of glutathione, i.e., glycine, L-cysteine (as L-cystine) and a glutamate source (which can, in turn, be provided in the form of glutamic acid or glutamine) plus a selenium containing amino acid such as selenomethionine or selenocysteine serving as a cofactor. The composition may also contain other amino acids, such as, for example, methionine, arginine, oxoproline, and the like.

Preferably, the compositions are pharmaceutical compositions in which the individual components, e.g., the selenium source and the glutathione precursor constituents are combined with a pharmaceutically acceptable carrier, solvent, emollient, surfactant, humectant, viscosity enhancer, or an emulsifier, etc.

Individual components of the compositions are described in the aforementioned Crum et al. (US 2012-0029082). Selenium is one of numerous trace metals found in many foods. In the compositions of this invention, selenium may be employed as one of several non-toxic, water soluble organic or inorganic selenium compounds capable of being absorbed through the mucosal membrane. Representative examples of the selenium source include, but are not limited to selenomethionine, selenite, methylselenocysteine, selenium nanoparticles, including salts, esters, anhydrides, tautomers or analogs, etc. of the individual selenium sources.

Representative examples of inorganic selenium compounds are aliphatic selenium metal salts containing selenium in the form of selenite or selenate anions. However, organic selenium compounds are also employable because they are normally less toxic than their inorganic counterparts. Other selenium compounds which may be mentioned by way of example include selenium cystine, selenium methionine, mono- and di-seleno carboxylic acids with about seven to eleven carbon atoms in the chain. Seleno-amino acid chelates are also useful. These selenium compounds may be considered for use in the present invention as selenium particles or salts thereof. Representative examples are known in the art. See Kojouri et al. "The Effects of Oral Consumption of Selenium Nanoparticles on Chemotactic and Respiratory Burst Activities of Neutrophils in Comparison with Sodium Selenite in Sheep," Biol Trace Elem Res. May 2012; 146(2): 160-166.

The glutathione precursor includes, individual components, e.g., L-glutamic acid, L-cystine and L-Methionine (as the L-cysteine source) and glycine, or one or more biological precursors thereof (e.g., glutamate [Glu] or glutamine [Gin] as a precursor of glutamic acid; cysteine [Cys], including modified cysteine derivatives such as N-acteylcysteine [NAC], as a source of L-cystine, etc.). Other usable forms of the GSH component compounds include, for example, salts, esters, anhydrides, tautomers or analogs of glutamic acid, cystine and glycine. The aforementioned components of the compositions of the instant invention can be administered simultaneously, sequentially or separately to a subject in need of such treatment.

All amino acids employed in this invention, except glycine which does not form optical isomers, are in the natural or L-form and are free form.

Although any ratiometric amounts of the individual components of the GSH precursor may be employed, it will be apparent to those skilled in the art that the end-stage ratio of L-glutamic acid to L-cystine to glycine in the novel compositions described herein is 1:0.5:1 (or 2:1:2). If an excess of any acid is used, it will presumably be of nutritional value or may simply be metabolized.

The question of whether an individual cell can make "too much" glutathione is discussed later in this exposition. However, suffice it to say that repletion of cellular glutathione, when synthesized from its constituents physiologically, in the step-by-step manner as has been evolutionarily perfected, should protect and monitor the optimal physiological glutathione within its reference range for the synthesis and quantification of cytosolic glutathione. Importation of the intact glutathione molecule into the cytosol (intracellular space) can disrupt this enzymatically regulated process and can lead to vestigiality of the synthesis enzymes which would be thrown into disuse, and its quantification-monitoring for glutathione would be thrown into uncertainty. Importation of the intact glutathione molecule could also lead to "reductive stress" because the glutathione quantification would not be enzymatically monitored and regulated.

As will be apparent to the skilled artisan, owing to the toxicity of the selenium compound, the dosage units for mammalian administration by any selected route will cater to avoiding treatment either with single or multiple dosages of the toxic compound and the dosage of the selenium compound will be adjusted so that the total delivery does not reach the toxic limit of 400 µg/day for adult humans (Institute of Medicine, Food and Nutrition Board. Dietary Reference Intakes: Vitamin C, Vitamin E, Selenium, and Carotenoids. National Academy Press, Washington, DC, 2000).

The recommended daily allowances for selenium as reported in The Pharmacological Basis of Therapeutics, 9th Ed., The McGraw-Hill Companies, 1996 are shown in Table 1 below:

**Table 1. Recommended daily allowances for selenium.**

| Subject | Age/Years | Dose/µg |
|---|---|---|
| Infants | 0.0-0.5 | 10 |
| | 0.5-1.0 | 15 |
| Children | 1.0-3.0 | 20 |
| | 4.0-6.0 | 20 |
| | 7.0-10.0 | 30 |
| Males | 11.0-14.0 | 40 |
| | 15.0-18.0 | 50 |
| | 19.0-24.0 | 70 |
| | 25.0-50.0 | 70 |
| | 51+ | 70 |
| Females | 11.0-14.0 | 45 |
| | 15.0-18.0 | 50 |
| | 19.0-24.0 | 55 |
| | 25.0-50.0 | 55 |
| | 51+ | 55 |
| Pregnant | - | 65 |
| Lactating | 1st six mo. | 75 |
| | 2nd six mo. | 75 |

The recommended daily dosage for humans therefore ranges from 10 to 75 µg per day depending on age. For animals the range may be generally higher but will, of course, depend upon the animal and its size.

The precise amount of the therapeutically useful compositions of this invention for daily delivery and the duration of the period of such delivery will depend upon the professional judgment of the physician or veterinarian in attendance. Numerous factors will be involved in that judgment such as age, body weight, physical condition of the patient or animal and the ailment or disorder being treated.

It is important for the practice of this invention that the selenium as employed in the composition be capable of transport through the mucosal membrane of the patient under treatment. For this reason, water insoluble selenium compounds are not generally useful.

For convenience, the term "selenium" is sometimes used hereinafter to include any of the various water soluble selenium products which can be transported through the mucosal membrane in the practice of this invention. It will be understood, however, that the particular forms of selenium compounds set forth herein are not to be considered limitative. Other selenium compounds, which exhibit the desired activity and are compatible with the other components in the mixture and are non-toxic, can be used in the practice of the invention. Many of them are available commercially.

Preferably, the selenium is provided with L-methionine (e.g., selenomethionine) or with L-cystine (e.g., selenocystine) or selenocysteine. The provision of selenium as the latter allows accomplishment of two goals simultaneously, (a) provision of the selenium co-factor; and (b) provision of an additional safe source of L-cysteine, the rate-limiting amino acid.

In fact, the amount of selenium precursor employed in the novel compositions is only enough to provide a catalytic quantity of the element to activate the glutathione system. The catalytic quantity of selenium precursor utilized in the compositions of this invention is such that it will produce either in one dosage unit or in multiple dosage units sufficient elemental selenium to promote the production and activation of glutathione. Typically, this will be at or near the recommended daily allowance of selenium for the individual mammal under treatment. This amount will be well below the toxicity limit for elemental selenium. By way of nonlimiting examples, a representative range of catalytic quantity of selenium is presented in the aforementioned Table 1, as shown to be effective based on the subject' s age.

### Compositions

This invention provides pharmaceutical compositions for use as defined in the appended claims. Such compositions comprise a therapeutically effective amount of combined L-glutamic acid, L-cystine (as the L-cysteine source), glycine and a selenium precursor in a pharmaceutically acceptable carrier. In a particular embodiment, the term "pharmaceutically acceptable" means one that is generally recognized as safe, approved by a regulatory agency of the federal or a state government or listed in the U.S. Pharmacopeia or other generally recognized pharmacopeia for use in animals, and more particularly in humans. The term "carrier" refers to a diluent, adjuvant, excipient, or vehicle with which the active compounds are administered.

The compositions which may be provided in bulk or dosage unit form are prepared in accordance with standard pharmaceutical practice and may contain excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Sterile liquids, such as water and oils, including those of petroleum, animal, vegetable or synthetic origin, such as peanut oil, soybean oil, mineral oil, and sesame oil may also be useful. The composition, if desired, can also contain minor amounts of wetting or emulsifying agents, coloring agents or buffering agents.

Buffering agents are sometimes used in the compositions of the invention to maintain a relatively constant hydrogen ion concentration in the mouth (pH about 7.5) or other point of entry. An appropriate buffering agent may be selected from numerous known reagents including, for example phosphate, carbonate and bicarbonate systems. Alpha-lactalbumin is useful because of its buffering properties. Additionally, it is non-toxic, water soluble and contains appreciable amounts of the required amino acids.

The compositions may also contain mucous membrane penetration enhancers and skin penetration enhancers such as sodium lauryl sulphate, sodium dodecyl sulphate, cationic surfactants such as palmitoyl DL carnitine chloride, cetylpyridinium chloride, non-ionic surfactants such as polysorbale 80, polyoxyethylene 9-lauryl either, glyceryl monolaurate, polyoxyalkylenes, polyoxyethylene 20 cetyl ether, lipids such as oleic acid, bile salts such as sodium glycocholate, sodium taurocholate and related compounds.

Examples of these suitable carriers are described in Remington's Pharmaceutical Sciences, Nineteenth Edition (1990), Mack Publishing Company, Easton, Pa. in Handbook of Pharmaceutical Excipients, published by The American Pharmaceutical Association and The Pharmaceutical Society of Great Britain (1986) and the Handbook of Water-Soluble Gums and Resins, ed. By R. L. Davidson, McGraw-Hill Book Co., New York, N.Y. (1980). Compositions and methods of manufacturing compositions capable of absorption through the mucosal tissues are taught in U.S. Pat. No. 5,288,497. They can be readily employed by the skilled artisan to devise methods of delivery other than those specifically described in this disclosure.

### Dosages

The compositions of the invention are most conveniently utilized in dosage units for oral administration. They may be used alone but are preferably provided as tablets, suitably sublingual tablets. Such tablets may be prepared in one a day form or for intermittent use throughout the day, for example every three hours.

The tablets will typically weigh from about 0.5 to 5 grams and will contain a therapeutically effective amount of the essential ingredients together with the selected vehicle. "Therapeutically effective," as used herein, means the amount of the composition which is sufficient to achieve the desired result, i.e., enhancement of the immune system. It means that the immune system is more effective in combating infection after treatment than it was before treatment.

A particular advantage of the compositions for use of the invention is that they can be provided in a number of different forms and at dosage levels appropriate to the individual mammal being treated. For example, tablets, elixirs, solutions, emulsions, lotions, powders, capsules and other forms can be provided for one a day treatment or successive treatments on the same day for animals or humans whether male or female, whether infant, adolescent or adult. The defining feature of this advantage is the amount of selenium and sulfur precursor utilized since the other components are essentially non-toxic.

Referring to the table above, tablets and other forms of the immunoenhancing compositions can be prepared to provide any quantity of elemental selenium from less than 1 to 7.5 µg. For example, a tablet containing 10 µg of selenium methionine is capable of delivering 4µg of elemental selenium, and 7.5 µg of selenium methionine is capable of delivering 3µg of selenium. Tablets may be given several times per day to achieve the desired immune enhancing effect.

A one a day tablet weighing two grams may contain 200 mg or more of the composition (10% by weight). A similar tablet intended to be used every four hours may contain 50 mg to 100 mg or more of the therapeutically effective composition. Equivalent amounts of carrier and active components will be utilized in other compositions designed for other methods of administration.

The aforementioned compositions and combinations may include suitable additives and further pharmaceutical ingredients. Examples of such additives include, but are not limited to, for example, coenzyme Q10 (CoQ10), ubiquinone, 7-keto dehydroepiandosterone (7-keto DHEA), N-acetyl-cysteine, magnesium orotate or a combination thereof. See Hastings et al. (US patent No. 6,368,617) and Richardson et al. (US patent No. 6,207,190).

### Delivery agents

As indicated above, the presently preferred method of delivery for the compositions is oral, topical, sublingual buccal or epithelial. It is convenient to provide dosage units for such delivery in the form of pills, lozenges or tablets such as gelled tablets which will slowly dissolve in the mouth. Furthermore, for topical delivery, the formulation may be in the form which would be appropriate to the skin, such as lotions, unguents, emollients, creams, etc.

Nasal delivery will typically be accomplished by sprays or drops. Suppositories will be useful for rectal or vaginal delivery.

### Methods for reducing the adverse effects of statin compounds (not part of the invention)

Although statin compounds are recognized to have a wide margin window of safety, they are laden with a host of side effects. See, Richardson et al. US patent app. pub. No. 2011-0104137. Recent reports of 50 consecutive cardiology patients on statin drug therapy for an average of 28 months revealed possible adverse effects related to statin drug use. See, Langsjoen et al. "Treatment of statin adverse effects with supplemental Coenzyme Q10 and statin drug discontinuation," Biofactors, 2005; 25(1-4):147-52 (PMID: 16873939). These side effects include myalgia, fatigue, dyspnea, memory loss and peripheral neuropathy. It was thought that the side effects are due to a decrease in the production of a lipophilic enzyme known as coenzyme Q10 (CoQ10) also known as ubiquinone, due to the inhibition of mevalonate synthesis by the statin. These side effects offer a serious impediment to prolonged use of statin drugs. In addition these side effects may limit the most recent guidelines of the National Cholesterol Education Program which called for more aggressive use of statins to lower LDL levels to at least less than 100 mg/dl for patients at moderate or high risk of cardiovascular disease and to less than 70 mg/dl for patients at high risk of vascular disease. See, Guthrie et al. "How safe is aggressive statin therapy?" Prog Cardiovasc Nurs. 2006 Summer; 21(3): 140-5. These guidelines have been recommended because of the continued correlation of higher incidence of death from vascular disease at higher levels of cholesterol. Reducing the total amount of LDL does not appear to be the total answer to avoiding atherosclerosis. Thus, the concept that the amount of oxidized LDL may actually be a more important factor than the total amount of LDL in terms of contributing to artery disease is beginning to emerge.

Brown et al. (US 4,933,165), teaches adding CoQ10 to replace the loss of CoQ10 that occurs with the use of the HMG-CoA reductase inhibitor. Other prior art references teach direct administration of glutathione compound (in the form of the tri-peptide) to attenuate the side effects of statins. See, Guilford et al. (US patent app. pub. No. 20140141071), which teach liposomal vesicles comprising glutathione. In contrast, an embodiment of the instant invention is directed to use of the aforementioned composition comprising the glutathione precursor compounds (e.g., glycine, L-cystine and glutamate source) and the selenium source to enhance the effect of maintaining cholesterol and LDL in the biochemically reduced state. By maintaining the reduced state, that is avoiding the oxidation of cholesterol or the oxidation of LDL, the most beneficial effect of reduced glutathione and a statin working together is achieved. Further, the goal of lessening the formation of atherosclerosis will be achieved using a lower dose of the statin drug and thereby minimizing the side effects of the statin. Moreover, in contrast to the current goal of statin therapy, i.e., lowering of LDL levels to lessen the progression of atherosclerosis, the compositions of the instant invention attenuate the progression of atherosclerosis by lessening the formation of oxidized LDL, and facilitating the function of HDL. As has been recognized in the art, decreased oxidized LDL is advantageous in avoiding atherosclerosis.

It should be remembered that liposomally penetrating the cell wall with intact glutathione presents a risk to the substrate-specific enzymes which have been evolutionarily perfected to proceed step-by-step in the synthesis of glutathione. Further, liposomally penetrating the cell wall with intact glutathione carries the more immediate risk of exceeding the optimal levels of glutathione normally monitored by each cell's shut-down mechanism, and creating an immunological reductive stress impediment to the immune system. (See Narasimhan M and Rajasekaran NS "Reductive potential - A savior turns stressor in protein aggregation cardiomyopathy," Biochimica et Biophysica Acta, 1852 (2015) 53-60.)

Herein described are methods for lowering the adverse effects of statin therapy by using the aforementioned compositions optionally together with coenzyme Q10 (CoQ10) or ubiquinone. The net effect of the composition is to reduce the oxidation of cholesterol, which will result in an improved avoidance of vascular occlusion and also avoid the complications associated with depleted CoQ10 levels.

In a still further embodiment there is described a method of treatment to minimize the adverse side effects of a drug therapy, e.g., statin therapy, comprising administering to a subject in need of such treatment, an effective amount of at least the above-mentioned composition, by which the statin dosage may be lowered and hence the adverse side effects of the statin could be lowered. The drug therapy may be, for example, a therapy for hypercholesterolemia, a therapy for hyperlipidemia, corticosteroid therapy or cancer chemotherapy. One embodiment of the invention can be used to re-establish the immune system after its compromise from chemotherapy.

In a still further embodiment there is described a method of treatment of a side effect of a drug therapy, e.g., statin therapy, comprising administering to a subject in need of such treatment an effective amount of at least the above-mentioned composition. The drug therapy may be, for example, a therapy for hypercholesterolemia, a therapy for hyperlipidemia, corticosteroid therapy or cancer chemotherapy or to re-establish the immune system after its compromise from chemotherapy.

It should also be recalled that in major university study, "A novel cystine based antioxidant attenuates oxidative stress and hepatic steatosis in diet-induced obese mice." Experimental and Molecular Pathology. 2011 August: 91(1):419-428) the invention was found to have activated gene Nrf2, the master regulator of the immune system. Earlier the multi-functional Mechanisms of Action were described which demonstrated the ways that the invention can be an adjunct to foster reduced statin use or as a possible replacement for statins altogether.

According to another embodiment, there is described a method for statin replacement therapy comprising administering to a subject in need of such treatment an effective amount of each of the components of the aforementioned compositions, or salts, esters, anhydrides, tautomers or analogs thereof either simultaneously, sequentially or separately, optionally in association with one of more pharmaceutically acceptable additives. The compositions for use of the instant invention are advantageous over statins because of demonstrated reduced toxicity and also economically beneficial for long-term therapy (reduced costs). There are virtually no side effects and additionally, the compositions may be for use to reduce and/or reverse statin-induced sarcopenia.

In lipogenesis, it has been demonstrated that the invention activates Nrf2, which may be the reason for the invention's ability to reduce hypertriglyceridemia and, to some extent, hypercholesterolemia. In addition, other biomarkers have been established for this novel and innovative development. See the university research report: "A novel cystine based antioxidant attenuates oxidative stress and hepatic steatosis in diet-induced obese mice." Experimental and Molecular Pathology, 2011 August: 91(1):419-428 doi:10.1016/j.yexmp.2011.04.009. See biomarkers quoted herein which follows below:

"Nonalcoholic fatty liver disease (NAFLD) is the most common form of liver pathologies and is associated with obesity and the metabolic syndrome. Here, we investigated the molecular mechanisms by which a novel cystine based glutathione precursor with added selenomethionine (F1) prevents hepatic steatosis in a moderate high fat dietary model of NAFLD. Adult (8 weeks old), male apolipoprotein E (ApoE)-/- mice were fed with a normal diet (ND) or high fat diet (HFD), consisting of 21% fat and 0.21% cholesterol, with or without dietary supplementation of Fl (3 g/kg food) for 16 weeks. Compared with ApoE-/- mice fed with ND with or without F1, ApoE-/- mice fed with HFD exhibited significant weight gain, hepatomegaly, and increased serum cholesterol and triglycerides levels with no change in serum albumin levels. High resolution light and electron microscopy revealed micro-and macro-vesicular steatosis in ApoE-/- mice fed on a HFD. HFD-induced obesity also led to increased lipogenesis, oxidative stress, activation of c-Jun-NH(2)-terminal kinase (JNK) and p38 mitogen-activated protein kinase (MAPK), perturbation of the BAX/BCL-2 rheostat, hepatocyte apoptosis, and activation of caspases 9 and 3. F1 fully prevented the adverse effects of HFD on serum triglyceride levels, body and liver weights, and hepatic steatosis and substantially attenuated HFD-induced increase in lipogenesis, oxidative stress, kinase activation, apoptotic signaling, and hepatocyte ultrastructural abnormalities. These results demonstrate that administration of F1, a glutathione precursor, ameliorates HFD-induced hepatic steatosis in ApoE-/- mice and emphasizes the role of oxidative stress in diet-induced obesity and hepatic steatosis."

In the aforementioned methods, e.g., reduction of the side effects of statin therapy or replacement of statin therapy, each of the individual components of the compositions, i.e., the selenium source and the glutathione precursor compounds, are preferably of pharmaceutical grade and the glycine and L-cystine and L-glutamate source in the final composition is adjusted to attain an equimolar in vivo ratio of the three amino acid components. However, it is contemplated that the formulations for use of the invention comprising a glutamine source and the L-cysteine source (in the form of 2 L-cysteines in a disulfide L-cystine bond) and an additional L-cysteine source in the form of L-methionine and a glycine source may allow delivery of extra L-cysteine safely to cells, which L-cysteines are utilized in other metabolic functions.

In a further embodiment, there is described use of the aforementioned components of the compositions of the invention, one of its biological precursors or a salt, ester, anhydride, tautomer or analog thereof, optionally together with a carrier, suppository, emulsifier, or solvent in preparation of a medicament for treatment or reduction of the incidence of one or more side effects of statin therapy.

In a still further embodiment, there is described use of the aforementioned compositions of glutathione precursor and selenium compound, optionally together with one or more pharmaceutically acceptable additives in preparation of a medicament for treatment of one or more side effects of statin therapy.

The components of the present invention combination would be supplied in a single formulation comprising the glutathione precursors and the selenium source. Alternatively, the components of the present invention combination would be supplied in a single formulation comprising the glutathione precursors and CoQ10. Alternatively, the components could also be taken individually, but concurrently in their present pill, capsule, powder or liquid form in order to individualize the amount of each component to the needs of the individual. The individual dosages of the selenium compound and the glutathione precursor compounds have been detailed in the foregoing paragraphs and Table 1. For the statin compound, the dose may range from 0.10 to 80 mg/day in a single or divided administration. The dose of CoQ10 may vary from 25 mg to 1 g in single or divided doses. A typical CoQ10 dose for a normal adult (70 kg) would be 100 mg per day. Other agents, such as, N-acetyl cysteine, a-lipoic acid, vitamin C, vitamin E, silibinin, resveratrol etc. may also be added to the combination, as needed.

The dose used in the combination of statin and the composition comprising the glutathione precursors and the selenium compound is 10 mg of statin (e.g., simvastatin) once a day and 400 mg each of the glycine, L-cystine (as the L-Cysteine source), and glutamic acid source (e.g., glutamate and/or glutamine). The dosage of selenomethionine is adjusted in such a way that the dosage of selenium therein it would not exceed the recommended daily value. In a preferred embodiment, the combination would be formulated such that the dose of the statin compound is about 10 mg and the dose of the glutathione precursors as above with the elemental selenium in the composition is between 1µg to 7.5 µg, endpoints included.

In a related embodiment, herein described are methods for improving the pharmacological profile of a statin compound, comprising administering to a subject in need thereof a pharmacological composition comprising a glutathione precursor (e.g., glycine, L-cysteine and a glutamate source such as glutamine or glutamic acid, as detailed in the foregoing paragraphs) and a selenium source (e.g., selenocystine selenocysteine or selenomethionine). The pharmacological composition may optionally comprise a statin compound or other ingredients such as a cholesterol absorption inhibitor, a bile acid binding resin, or a fibrate.

Herein, the improved pharmacological profile could be increased efficacy of the statin compound or reduced toxicity of the statin compound or a combination of improved efficacy and reduced toxicity of the statin compound or the replacement of the statin with the instant invention.

The side effects associated with statin therapy could be observed in any clinical setting, e.g., during or following therapy of hypercholesterolemia or hyperlipidemia (dyslipidemia) with statins, during or following cancer therapeutics with statin compounds, during or following the therapy of dementia, during or following the treatment nuclear cataracts, etc.

### Methods for reducing blood cholesterol and/or low-density lipoproteins

In an alternative embodiment, the present invention provides use of the composition comprising the glutathione precursor amino acids and the selenium source in combination with a statin to ameliorate the oxidation of cholesterol, HDL and LDL and to lessen the risk of vascular accident. It has been found in the past that the lowering of cholesterol will result in a decreased risk of vascular accident, but there has been no previous recommendation of a substance or combination to reverse the oxidation state of lipids in the prevention of vascular accidents. Owing to their reductive potential, the compositions of the instant invention confer beneficial effects by lowering oxidized LDL in the blood. For other beneficial of effects of the instant invention including the activation of the immune regulatory gene Nrf2, see "A novel cystine based antioxidant attenuates oxidative stress and hepatic steatosis in diet-induced obese mice." Experimental and Molecular Pathology, 2011 August: 91(1):419-428 doi:10.1016/j.yexmp. 2011.04.009.

In a related embodiment, the composition of instant invention confers reduction of cholesterol and/or LDL without side effects of statins. As mentioned in the foregoing paragraphs, prolonged exposure to statin for lowering of blood cholesterol and/or LDL has been demonstrated to adversely increase risk of myopathy, increase liver transaminases leading to liver, renal and neurological toxicity, risk of incident diabetes mellitus, depletion of plasma coenzyme Q10 levels, and increase muscle pain. The beneficial effect of the composition of the instant invention in the management of the risk of statin toxicity, especially in patients with chronic diabetes or dyslipidemia, and superiority of the composition to the Statin model (with none of such adverse side effects associated with the statin model) was hitherto unknown in the medical literature.

Moreover, the pharmacological effect of the composition of the instant invention as a replacement of the statin model was unexpected. For example in a human clinical trial, the composition of the instant invention achieves greater scavenging of oxidized lipid products compared to N-acetyl cysteine (135% versus 1%). Reason: the instant invention physiologically provides more rate-limiting L-cysteine available in the cell together with the other constituents of glutathione.

### Methods for inhibiting pathogen replication and/or treatment of diseases (not part of the present invention)

The present disclosure is broadly concerned with techniques for chelating or ejecting Zn²⁺ from a zinc finger peptide which may be a part of viral proteins or viral RNA polymerases, for example, so as to inhibit viral replication. Broadly speaking, the methods described herein involve contacting the viral zinc-finger peptide with an effective amount of the aforementioned composition comprising a glutathione precursor and a selenium source (including salts of the amino acids which are in the precursor compound and/or the selenium source), and thereby causing the desired chelation or ejection. The methods may be conducted in vivo or in vitro. The art recognizes that zinc is an important co-factor in these proteins (see, Modrof et al. "Ebola Virus Transcription Activator VP30 is a Zinc-Binding Protein" Journal of Virology, March 2003, pp 3334-3338 and Esperante et al. "Fine Modulation of the Respiratory Sycytial Virus M2-1 Protein Quaternary Structure by Reversible Zinc Removal from its Cys3-His Motif." Biochemistry 2013, 52, 6779-6789). Accordingly, chelation of zinc from the affected cells effectively disrupts viral replication.

The methods described herein find utility in the control or treatment of a variety of viruses and viral diseases, such as HIV, polio, human coxsackie, SARS, rabies, human parainfluenza, measles, human respiratory syncytial, human hepatitis, Dengue, West Nile and Ebola. The aforementioned compositions may also be effective against malarial Plasmodium falciparum and Leishmania donovani parasites.

### Methods for treating erectile dysfunction (not part of the present invention)

Relating to blood circulation, herein described are methods for treating or reducing the incidence of erectile dysfunction (ED) in a subject in need thereof, comprising administering to said subject one or more of the aforementioned compositions comprising the glutathione precursor and the selenium source. In such embodiments, the glutathione precursor comprises glycine, L-cystine and a glutamate source which is glutamine or glutamic acid.

In a related embodiment, herein described are methods for treating or reducing the incidence of erectile dysfunction comprising administering to a subject in need thereof a composition as described herein comprising glutathione precursor and a selenium source and another drug for treating erectile dysfunction selected from the group consisting of Sildenafil (VIAGRA), tadalafil (CIALIS) and vardenafil (LEVITRA).

Embodiments described herein further relate to the novel interrelationship between the functions of L-cystine and L-selenomethionine and how the unique features of their functions play a vital role in the unexpected activation of the elusive gene Nrf2. Nrf2 is considered a life-preserving gene that is known to attenuate inflammation and oxidative stress. Nrf2 is an antioxidant and inflammation modulator gene, which works by inducing the transcription of "more than 250 genes that decrease the level of oxidative stress and several inflammatory mediators." See the September 23, 2010 Press Release entitled "Abbott and Reata Pharmaceuticals Announce Agreement to Develop and Commercialize Bardoxolone Methyl for Chronic Kidney Disease Outside the U.S."

The quality and extension of life is affected by the Immune System's ability to control inflammation and oxidative stress. Activating Nrf2 to support and have an ancillary role in maintaining the Immune System via modulation of inflammation and oxidative stress has been a long-sought-after goal of immunological research. Certain synthetic products have been studied, e.g., Bardoxolone methyl and DH404. There are numerous side effects associated with these synthetic agents. In contrast, the utility of naturally occurring bodily amino acids, L-cystine a L-glutamate source, glycine and L-selenomethionine (either as a replacement therapy or as an adjuvant to synthetic agents), to accomplish the activation of Nrf2, is advantageous. It has been demonstrated that L-cystine and L-selenomethionine have a novel and unexpected role in the synthesis of glutathione. See Sinha et al. ("Inhibition of apoptotic signaling in spermine-treated vascular smooth muscle cells by a novel cysteine based glutathione precursor," Cell Biology International. 2010); Indrani et al. ("Effects of a novel Cystine based glutathione precursor on oxidative stress in vascular smooth muscle cells," Am J Physiol. 2010); and Indrani et al. ("A novel Cystine based antioxidant attenuates oxidative stress and hepatic steatosis in diet-induced obese mice," Experimental and Molecular Pathology, 2011).

The role of L-cystine in glutathione synthesis was novel and unexpected because L-cystine is already oxidized, and it previously seemed counter-intuitive to expect that a presently oxidized molecule could play a vital role in the mechanism of antioxidation. In fact, many scientists have even categorized L-cystine primarily as only a biomarker of oxidative stress. See, for example, Dhawan et al. ("The role of plasma aminothiols in the prediction of coronary microvascular dysfunction and plaque vulnerability," Atherosclerosis, 2011); Patel et al. ("Oxidative stress is associated with impaired arterial elasticity," Atherosclerosis, 2011). For such reasons, L-cystine was overlooked for many years as having a major role in the synthesis of the major antioxidant glutathione. Further, L-cystine has been described as merely "used up L-cysteine. However, the disulfide bond of L-cystine is biochemically decoupled by thioltransferase. The pleiotropy of the invention gives it multi-functional benefits.

In contrast, the embodiments described herein proceed contrary to wisdom in the art regarding the oxidative role of L-cystine. As further detailed in Sinha-Hikim et al. (2010), "F2 (N-acetylcysteine fortified with the same amount of selenomethionine like the Fl compound comprising the composition of the instant application) is less effective in ameliorating spermine-induced reduction in GSH levels and perturbation in cellular morphology. See also US RE39,734 and RE42,645. Thus it is conceivable that cystine replacing cysteine in F1 formulation may play an important role in F 1-mediated protection of vascular smooth muscle cell (VSMC) against spermine-induced injury. A priori, it seems paradoxical that cystine, the oxidized form of cysteine, can lead to an enhanced redox state. This seeming paradox is unraveled in the revelation of the pleiotropic nature of Cystine/cysteine with Cystine, functioning as a cysteine carrier, whereby cysteine in the plasma undergoes auto-oxidation to a stable and relatively inert Cystine. The "stable cysteine" now in its oxidized form as Cystine, can be taken up from the extracellular space and decoupled intracellularly back to two cysteines, via substrate-specific enzymes, oxidoreductase and thioltransferases, supplying the intracellular cysteine necessary for glutathione and protein biosynthesis. See Zhu et al. (2008). In this context, it is worth noting that in an in vitro setting we find that Fl is even more effective than NAC in preventing spermine-induced VSMC apoptosis through suppression of JNK and nitric oxide-mediated intrinsic pathway signaling. See Sinha-Hikim et al. (2010).

Thus, it is contemplated that cystine replacing cysteine in F1 formulation may play an important role in Fl-mediated protection of HFD-induced fatty liver. See Indrani et al. "A novel cystine based antioxidant attenuates oxidative stress."

One skilled in the art understands that L-cystine is a metabolite amino acid in the L-cystine is a metabolite amino acid in the catabolism of protein. It is found in certain protein foods, such as lean beef, clams, veal, turkey, chicken, fish, crabs, lobster, et al. L-cystine is a compound of two amino acids, L-cysteine and L-cysteine, which have auto-oxidized into a unity via a disulfide bond which unites these two L-cysteines into the new chemical molecule. L-cystine has radically different properties from the L-cysteine molecules from which it is formed. L-cystiene can also be transsulfurated from L-methionine. It has a vital role in the metabolism of Vitamin B6. It was previously thought that because L-cystine is relatively stable, by virtue of its disulfide bond, that it was inactive, effete, oxidized or "used-up." See, Emory University Public Press Release April 4, 2011 "Measuring oxidative stress can predict risk of atrial fibrillation."

In contrast to cysteine, which has little bodily physiological and biochemical functions, L-cystine, in addition to the above, is important to the formation of insulin, sperm cells, skeletal muscle, connective tissues, hair and certain enzymes. Further, the disulfide bond serves many vital bodily biochemical and physiological functions (see list below). The use and role of the auto-oxidation in L-cystine is an evolutionary adaptation of major significance. However, the scientific literature has only peripherally touched upon its importante. Rather than emphasizing its significance, the resulting auto-oxidized molecule, L-cystine, has often been classified as "used-up cysteine" or classified exclusively as a biomarker of oxidative stress, and as an indication of a pathological oxidized state. See Dhawan et al. (above); Patel et al.'s article entitled "Oxidative stress is associated with impaired arterial elasticity."

Research has recently demonstrated that L-cystine exemplifies a pleiotropic paradox, and its role is vital in the synthesis of glutathione and certain other concomitant but unexpected results, such as the activation of the imorttant gene Nrf2. See the aforementioned publications by Sinha---Hikim et al. On closer examination, and upon extensive university research, other dimensions to L-cystine have been verified. It has been documented in the literature and in university research that L-cystine is stable and neutral and water insoluble, as compared to L-cysteine, which is highly oxidizable and somewhat toxic to the body. See Janaky et al. ("Mechanisms of L-cysteine Neurotoxicity," Neurochemical Research. Vol. 25. Nos 9/10, 2000, pp 1397-1405); Dilger et al. "Excess dietary L-Cysteine, but not L-cystine, is lethal for chicks but not for rats or pigs," Journal of Nutrition, 2007 Feb;137(2):331---8); Crum et al. Presentation before American Chemical Society, August 21, 2007 entitled "Sulfenic acid, sulfinic acid, sulfonic acid."

Although L-cysteine is the crucial and most valuable functional detox moiety of glutathione (considered the body's master antioxidant), getting the L-cysteine into the intracellular space, where it could enter into the glutathione synthesis chain, was for a long time considered a scientific enigma. When a highly oxidizable molecule such as L-cysteine, which has toxic features, is also important for the physiological synthesis of glutathione, it can be comprehended that nature has adapted an evolutionary advantage to auto-oxidation of that molecule (L-cysteine) for its safe carriage to the intracellular milieu where it can be utilized for the physiological synthesis of glutathione. There have been other methods tried to get the highly oxidizable L-cysteine into the cytosol, but with limited results. The synthetic ester, N-acetylcysteine, has been used by scientists to reduce the high reactivity and high oxidizability of the solo L-cysteine, so as to enable it to reach the intracellular glutathione synthesis chain with less reactivity and less oxidizability. Large protein molecules from non-denatured whey have also been used in an effort to keep the highly reactive, highly oxidizable but rate-limiting L-cysteine "in check" until it could be decoupled so as to enter the intracellular space of the glutathione synthesis milieu.

The inventor of the instant application utilized the advantage of L-cystine's disulfide bond as the safe physiological carrier of L-cysteine as the method to accomplish this vital L-cysteine delivery role. Upon arrival at the cell wall, the tenacious disulfide bond of L-cystine is decoupled by substrate-specific enzymes, oxidoreductase, and thioltransferase, at the cell membranes and in the cytosolic milieu. The decoupling of the disulfide bond permits the released, free form L-cysteine to be available for incorporation into the reducing cytosolic media of the intracellular environment. Also present in the intracellular space is the substrate specific gamma-glutamylcysteine synthetase, readily available to catalyze a unity or L-cysteine to L-glutamic acid. Scientists had not realized or formulated the diverse physiological and biochemical potential of L-cystine in glutathione synthesis because it was widely perceived that the disulfide bond was essentially "fixed" or "irreversible." A typical comment or conclusion was that "Cystine is not suitable as an intracellular delivery agent (for L-cysteine) because of its marked insolubility." (P. 317 Methods in Enzymology, Volume 143.) Misconceptions have been made in limiting the functions of L-cystine to only a measurement or biomarker for oxidative stress. Attempts have been made to force a parallel interpretation of intracellular glutathione to extracellular L-cystine, because they both contain the sulfhydryl radical and are active in various redox functions. The sulfhydryl group in free form L-cysteine functions with different properties when it is in a solo amino acid as compared to when its sulfhydryl group is a moiety of glutathione.

In summary, solo L-cysteine has different, complex and paradoxical functions for its sulfhydryl that distinguish it from the sulfhydryl functions when it is a moiety of glutathione. A recent study has interpreted results that need further clarification. See, Patel et al., (Oxidative Stress is associated with impaired arterial elasticity." Atherosclerosis. 2011) which states "Non---free radical oxidative stress was assessed as plasma oxidized and reduced amino-thiol levels (cysteine/cysteine, glutathione/GSSG) and their ratios (redox, potentials), and free radical oxidative stress as derivatives of reactive oxygen metabolites (dROMs)."

### L-cystine and the evolutionary advantage of auto-oxidation

The disulfide bond is one of the strongest in biochemistry, with many evolutionary advantages and physiological functions (see below). It appears dormant and inactive (but in reality it could be viewed as a "sleeper" with latent and resurgent potential. The disulfide bond, while being a safe physiological carrier of L-cysteine, is also active as a bodily protector against the ravages of the high oxidizability of solo L-cysteine and allows the L-cysteine to be recycled, not only for the synthesis of glutathione, but also for the later intermediate role in the activation of gene Nrf2. The disulfide bond of L-cystine also serves an important scavenger function in forming an electron exchange coupling relationship embracing other solo L-cysteines. L-cystine is the only amino acid that is difficult to quantify in the laboratory in a solo state, because of its quantum-like coupling relationship with L-cysteine. The two molecules are practically inseparable, quantitatively speaking. The coupling relationship with the disulfide bond also serves a scavenger and recycling function for the rampant L-cysteine, as well as a bodily protective function. This coupling property provides an additional carrier capacity for more L-cysteines to be delivered into the intracellular glutathione synthesis chain, all the while providing additional protection from the oxidizability of free form L-cysteine. The element sulfur has an affinity and attraction to its own kind.

### Summary of L-cystine Functions

1. Activation of Nrf2.
2. Role in glutathione synthesis.
3. Carrier role for L-cysteine.
4. Bodily protection from high oxidizability, high reactivity of solo L-cysteine.
5. Auto-oxidation has a re-cycling role for L-cysteine.
6. Scavenger role to retrieve free-form L-cysteines, while maintaining these highly active amino acids in a neutral, nonpolar state.
7. Redox role with L-cysteine.
8. Stability: neutral valence benefits.
9. Strong bridge in insulin.
0. Strong bridge in spermatozoa.

Potential side effects (can be minimized): L-cystine's role in the extremely rare genetic vulnerability to cystinosis is well documented. Patients should consult their health professional if they have a genetic inability to metabolize L-cystine or have an allergie vulnerability to L-cystine or have a condition where L-cystine could be adverse.

When L-cystine was researched in disease states in major university centers, studies resulted in the elevation of bodily glutathione (RE42645). However, what was unexpected was its activation of the elusive gene Nrf2 and in lipogenesis. Although glutathione has long been viewed as "the Master Antioxidant," it also now appears to be a "gateway" to the immune system. It was not expected that glutathione could activate and work in the place of this vital Nrf2 gene. Years of university and pharmaceutical research have been devoted to studies for the activation of this life-saving gene, Nrf2.

L-methionine, as selenomethionine, has been discovered to be the third effective and safe carrier of L-cysteine. Selenomethionine carries L-cysteine via its property of transsulfuration, a metamorphosis-like process, changing its structure into L-cysteine with the release of selenium. The process can be analogized to a caterpillar's metamorphosis into a butterfly. The L-cysteine is the important rate-limiting molecule for the synthesis of glutathione, and glutathione, in turn, is the gateway to the activation of Nrf2. Among its many advantages, both of the components of selenomethionine (Selenium and L- methionine) are utilizable: (1) the methionine becomes an additional L-cysteine, a major metabolic pathway; (2) the selenium is an important co-factor for glutathione to function after it has been synthesized.

Without selenium, glutathione could not function after it has been synthesized. Selenomethionine is nature's alternate, equally important backup mechanism to replenish bodily glutathione by providing another source of L-cysteine. Nature needs a continuous supply of L-cysteine so the replenishment of glutathione can go forward, which, in turn, as stated, activates Nrf2 in lipogenesis. There is at least one condition under which L-cystine, as a physiological carrier of L-cysteine cannot supply L-cysteine sufficiently. That is, when an individual has a genetic tendency to calculi formation (cystinosis) from the disulfide bond provided by L-cystine.

Referring again to the need for a continuous supply of L-cysteine: there are two conditions where L-methionine as aphysiological carrier ofL-cysteine may not provide sufficient L-cysteine: (1) where there is liver disease and consequently the hepatic cells are unable to perform the transsulfuration; (2) where there is genetic tendency to homocysteine formation together with a concomitant deficiency in folic acid/Vitamin Bl2-if the deficiency in folic acid/Vitamin Bl2 is left unaddressed.

Bearing upon the two above conditions, individuals, who are genetically susceptible to calculi formation from L-cystine's disulfide bond, can be provided with the needed L-cysteine for glutathione synthesis by the selenomethionine route. This alternate, equally important metabolic route can supply additional valuable L-cysteine for the glutathione synthesis template. Nature adapted provisions to accommodate the need for alternatives and diversity and evolutionarily filled the gap with the selenomethionine process. It is not only glutathione's synthesis that needs a constant supply of L-cysteine, but also the unexpected activation of Nrf2 indirectly needs L-cysteine. Nrf2 can be down regulated and overwhelmed during periods of oxidative stress without the availability of the rate-limiting L-cysteine to replenish glutathione. The activation of Nrf2 is interrelated to an unexpected finding associated with glutathione's replenishment. The sulfur amino acids are delivered in granulated form as a combination of free form amino acid powder form from the manufacturer. The powder form total adult daily ingestion of sulfur amino acids is recommended not to exceed 1.6 to 3.2 grams per day. The proportion of the selenomethionine which represents elemental selenium should be taken into account, using U.S. Department of Agriculture, Food & Drug Administration recommendations as the Guide Line.

The daily adult dosage of selenium from all sources should not exceed 200 micrograms per day (see chart). Some suggested combinations are hereby presented. They are not to be considered a limitation since many apparent variations are possible without departing from the spirit or scope of the invention, and mixtures can be prepared for elixirs, pills, pellets, capsules or powder.

### Specific embodiments

A few of the many embodiments encompassed by the present description are summarized in the following numbered paragraphs. The numbered paragraphs are self-referential. In particular, the phase "in accordance with any of the foregoing or the following" used in these paragraphs refers to the other paragraphs. The phrase means in the following paragraphs embodiments herein disclosed include both the subject matter described in the individual paragraphs taken alone and the subject matter described by the paragraphs taken in combination. In this regard, the purpose in setting forth the following paragraphs to describe various aspects and embodiments particularly by the paragraphs taken in combination. That is, the paragraphs are a compact way of setting out and providing explicit written description of all of the embodiments encompassed by them individually and in combination with one another. As such, any subject matter set out in any of the following paragraphs, alone or together with any other subject matter of any one or more other paragraphs, including any combination of any values therein set forth taken alone or in any combination with any other value set forth, may be considered as being described. but not always a part of the present invention.

### Formulations/compositions (not part of the present invention)

Composition 1. A composition comprising a glutathione (GSH) precursor and a selenium source.

Composition 2. The composition in accordance with the foregoing or the following, wherein the glutathione precursor comprises glycine, L-cystine and a glutamate source.

Composition 3. The composition in accordance with the foregoing or the following, wherein the glutathione precursor comprises glycine, L-cystine and glutamate.

Composition 4. The composition in accordance with the foregoing or the following, wherein the glutamine source is glutamate (Glu) or glutamine (Gin).

Composition 5. The composition in accordance with the foregoing or the following, which is a pharmaceutical composition comprising a carrier, a solvent, an excipient, a surfactant or an emollient and optionally further comprising an additional pharmaceutical agent.

Composition 6. The composition in accordance with the foregoing or the following, wherein the selenium source is selenonomethionine, selenite, methylselenocysteine, or selenium nanoparticles.

Composition 7. The composition in accordance with the foregoing or the following, further comprising an additional pharmaceutical agent which is N-acetylcysteine, vitamin C, vitamin E, a-lipoic acid, folic acid, vitamins B6 and B12, silibinin, resveratrol or a combination thereof.

Composition 9. The composition in accordance with the foregoing or the following, further comprising a third medicament which is a cholesterol absorption inhibitor, a bile acid binding resin, or a fibrate.

Composition 10. A combination comprising at least two of the aforementioned compositions.

### Kits (not part of the present invention)

Kit 1. A kit comprising, in one or separate compartments, a glutathione (GSH) precursor and a selenium source, optionally together with an excipient, carrier or oil.

Kit 2. The kit in accordance with any of the foregoing or the following, comprising the glutathione precursor in one compartment and a selenium source in another compartment.

Kit 3. The kit in accordance with any of the foregoing or the following, comprising an additional pharmaceutical agent which is N-acetylcysteine, vitamin C, vitamin E, a-lipoic acid, folic acid, vitamins B6 and B12, silibinin, resveratrol or a combination at least two of the additional agents.

Kit 3. The kit in accordance with any of the foregoing or the following, further comprising instructions for formulating a composition comprising said glutathione (GSH) precursor and a selenium source.

Kit 4. The kit in accordance with any of the foregoing or the following, further comprising instructions for using the components, either individually or together, for the treatment of hyperlipidemia and/or hypercholesterolemia.

Kit 5. The kit in accordance with any of the foregoing or the following, further comprising instructions for using the components, either individually or together, for the treatment of the side effects of statin therapy.

Kit 6. The kit in accordance with any of the foregoing or the following, further comprising instructions for using the components, either individually or together, for the treatment of the side effects of statin therapy which is rhabdomyolysis, headache, joint pain, fever, muscle pain, back pain, abdominal cramping, sleep disorder, rhinitis, sinusitis, stimulation of coughing reflex, dizziness and/or fatigue.

### Compositions for use in treatment

Treatment 1. A composition for use in a method of treating hyperlipidemia and/or hypercholesterolemia in a subject in need thereof comprising applying the composition comprising a selenium source, a glutamate source, L-cystine and glycine in combination with a statin.

Treatment 3. The composition for use in accordance with any of the foregoing or the following, wherein said composition additionally comprises a pharmaceutically acceptable carrier, excipient, emollient, surfactant or solvent.

Treatment 4. The composition for use in accordance with any of the foregoing or the following, wherein said composition is a pharmaceutical composition for oral administration, topical administration, nasal administration, sublingual administration, buccal administration, anal administration or vaginal administration.

Treatment 5. The composition for use in accordance with any of the foregoing or the following, wherein said subject is a human or a non-human mammal.

Treatment 9. The composition for use in accordance with the foregoing or the following, wherein the statin compound is atorvastatin (e.g., LIPITOR, TORVAST), cerivastatin (e.g., LIPOBAY, BAYCOL; which were withdrawn from market in August 2001 due to the risk of rhabdomyolysis), fluvastatin (e.g., LESCOL, LESCOL XL), lovastatin (e.g., MEVACOR, ALTOCOR, ALTOPREV), mevastatin (e.g., COMPACTIN), pitavastatin (e.g., LIVALO, PITAVA), pravastatin (e.g., PRAVACHOL, SELEKTINE, LIPOSTAT), rosuvastatin (CRESTOR), simbastatin (e.g., ZOCOR, LIPEX), or a combination thereof.

Treatment 10. The composition for use according to foregoing or following, further comprising administering N-acetylcysteine, vitamin C, vitamin E, a-lipoic acid, folic acid, vitamins B6 and B12, silibinin, resveratrol or a combination thereof.

Treatment 75. A composition for use in a method for the treatment or reducing the incidence of hypercholesterolemia in a subject in need thereof, comprising a glutathione precursor and a selenium source in combination with a statin.

### Examples

The following examples are given by way of illustration only and are not to be considered a limitation since many apparent variations are possible.

Example #1: The patient, an 82 year old male, was placed on the Fl formulation (comprising the aforementioned glutathione precursor and selenium source in the form of PROIMMUNE^{®} 200) at a dosage of 3.2 grams per day for a period of over six months. As was shown in his medical laboratory reports, the patient's blood triglyceride, LDL, vLDL, and cholesterol levels were all at excellent levels, which were roughly the same as an average 18 year old male.

Example #2: (not part of the invention) The patient, a male aged approximately 81 years, used the Fl formulation (comprising the aforementioned glutathione precursor and selenium source in the form of PROIMMUNE^{®} 200) at dosage range of 1.6 to 3.2 grams per day for eight months. The patient had not previously used any drug (approved or otherwise) for treating erectile dysfunction. No other products for improving erectile performance were used during this period. The patient reported improvement in muscle strength. Surprisingly, the patient was able to ejaculate semen and had improved erectile performance after using the Fl formulation.

Another patient (age: approximately 72 years), also reported similar physiological effects after about eight months of continued use of the aforementioned Fl formulation.

Example #3: The medical chart for an individual shows blood test results for May 2, 2014 and for August 12, 2014. This individual was on a daily dose of the F1 (PROIMMUNE ) formulation described herein. In May, his values for total cholesterol, HDL cholesterol and LDL cholesterol respectively were 131, 52 and 67. In August, his values for total cholesterol, HDL cholesterol and LDL cholesterol respectively were 133, 53 and 66. Adult LDL levels below 100 mg/dl are considered optimal. These results demonstrate two things: the effectiveness of the Fl formulation and its continued effectiveness over time.

Although the description above contains many specifics, these should not be construed as limiting the scope of the invention, but as merely providing illustrations of some of the presently preferred embodiments of this invention. Thus, the scope of this invention should be determined by the appended claims.

## Claims

1. A composition for use in combination with a statin compound in a method for treating hypertriglyceridemia, hypercholesterolemia, and/or hyperlipidemia in a subject in need thereof, wherein the composition comprises a selenium source and a glutathione precursor, which is a mixture of a glutamate source, L-cystine, and glycine.

2. The composition for use according to claim 1, wherein the glutamate source is glutamine or glutamic acid.

3. The composition for use according to claim 1 or 2, wherein the glutamate source is L-glutamic acid, and wherein the L-glutamic acid, L-cystine, and glycine are in a ratio of 1:0.5: 1.

4. The composition for use according to any of claims 1-3, wherein the selenium source is selenomethionine, selenocystine, selenite, methylselenocysteine or selenium nanoparticles.

5. The composition for use according to any of claims 1-4, wherein the disease associated with hypertriglyceridemia, hypercholesterolemia and/or hyperlipidemia is coronary heart disease (CHD), stroke, peripheral vascular disease (PVD), hypertension, arteriosclerosis, diabetes, or a combination thereof.

6. The composition for use according to any one of claims 1-5, wherein the subject has previously undergone therapy with a statin compound.

7. The composition for use according to any one of claims 1-6, wherein the statin compound is atorvastatin (e.g., LIPITOR, TORVAST), cerivastatin (e.g., LIPOBAY, BAYCOL; which were withdrawn from market in August 2001 due to the risk of rhabdomyo lysis), fluvastatin (e.g., LESCOL, LESCOL XL), lovastatin (e.g., ME VAC OR, ALTOCOR, ALTOPREV), mevastatin (e.g., COMPACTIN), pitavastatin (e.g., LIVALO, PITAVA), pravastatin (e.g., PRAVACHOL, SELEKTINE, LIPOSTAT), rosuvastatin (CRESTOR), simbastatin (e.g., ZOCOR, LIPEX), or a combination thereof.

8. The composition for use according to claim 6, wherein the patient is initially under statin therapy prior to administration of said composition and the dosage of statin compound is lowered after the administration of said composition.

9. The composition for use according to claim 6, wherein said method comprises the administration of said composition with a third medicament that is a cholesterol absorption inhibitor, a bile acid binding resin, or a fibrate.

10. The composition for use according to any one of claims 1-9, wherein the composition is for oral, nasal, topical, epidermal, buccal, vaginal or rectal administration.

## Patentansprüche

1. Zusammensetzung zur Verwendung in Kombination mit einer Statinverbindung in einem Verfahren zur Behandlung von Hypertriglyceridämie, Hypercholesterinämie, und/oder Hyperlipidämie bei einer Person, die dies benötigt, **wobei** die Zusammensetzung eine Selenquelle und eine Glutathionvorstufe, die eine Mischung aus einer Glutamatquelle, L-Cystin und Glycin ist, umfasst.

2. Zusammensetzung zur Verwendung gemäß Anspruch 1,
**wobei** die Glutamatquelle Glutamin oder Glutaminsäure ist.

3. Zusammensetzung zur Verwendung gemäß Anspruch 1 oder 2,
**wobei** die Glutamatquelle L-Glutaminsäure ist, und
**wobei** die L-Glutaminsäure, L-Cystin und Glycin in einem Verhältnis von 1:0,5:1 vorliegen.

4. Zusammensetzung zur Verwendung gemäß einem der Ansprüche 1-3,
**wobei** die Selenquelle Selenomethionin, Selenocystin, Selenit, Methylselenocystein oder Selen-Nanopartikel ist.

5. Zusammensetzung zur Verwendung nach einem der Ansprüche 1-4,
**wobei** die Krankheit, die mit Hypertriglyceridämie, Hypercholesterinämie und/oder Hyperlipidämie assoziiert ist, eine koronare Herzerkrankung (KHK), ein Schlaganfall, periphere Gefäßerkrankung (PVD), Bluthochdruck, Arteriosklerose, Diabetes oder eine Kombination davon ist.

6. Zusammensetzung zur Verwendung nach einem der Ansprüche 1-5,
**wobei** die Person zuvor einer Therapie mit einem Statinpräparat unterzogen wurde.

7. Zusammensetzung zur Verwendung nach einem der Ansprüche 1-6,
**wobei** die Statinverbindung Atorvastatin (z.B. LIPITOR, TORVAST), Cerivastatin (z.B. LIPOBAY, BAYCOL; die im August 2001 auf Grund der Gefahr einer Rhabdomyolyse vom Markt genommen wurden), Fluvastatin (z.B., LESCOL, LESCOL XL), Lovastatin (z. B. ME VAC OR, ALTOCOR, ALTOPREV), Mevastatin (z. B. COMPACTIN), Pitavastatin (z. B. LIVALO, PITAVA), Pravastatin (z. B. PRAVACHOL, SELEKTINE, LIPOSTAT), Rosuvastatin (CRESTOR), Simbastatin (z. B. ZOCOR, LIPEX) oder eine Kombination davon ist.

8. Zusammensetzung zur Verwendung nach Anspruch 6,
**wobei** der Patient vor der Verabreichung der Zusammensetzung zunächst einer Statintherapie unterzogen wird, und die Dosis der Statinverbindung nach Verabreichung der Zusammensetzung verringert wird.

9. Zusammensetzung zur Verwendung gemäß Anspruch 6,
**wobei** das Verfahren die Verabreichung der Zusammensetzung mit einem dritten Medikament umfasst, das ein Cholesterinabsorptionsinhibitor, ein Gallensäure-bindendes Harz oder ein Fibrat ist.

10. Zusammensetzung zur Verwendung nach einem der Ansprüche 1-9,
**wobei** die Zusammensetzung für die orale, nasale, topische, epidermale, bukkale, vaginale oder rektale Verabreichung bestimmt ist.

## Revendications

1. - Composition pour une utilisation en combinaison avec un composé de statine dans une méthode de traitement de l'hypertriglycéridémie, de l'hypercholestérolémie et/ou de l'hyperlipidémie chez un sujet en ayant besoin, la composition comprenant une source de sélénium et un précurseur de glutathion, qui est un mélange d'une source de glutamate, de L-cystine et de glycine.

2. - Composition pour l'utilisation selon la revendication 1, dans laquelle la source de glutamate est la glutamine ou l'acide glutamique.

3. - Composition pour l'utilisation selon l'une des revendications 1 et 2, dans laquelle la source de glutamate est l'acide L-glutamique, et dans laquelle l'acide L-glutamique, la L-cystine et la glycine sont dans un rapport de 1:0,5:1.

4. - Composition pour l'utilisation selon l'une quelconque des revendications 1 à 3, dans laquelle la source de sélénium est la sélénométhionine, la sélénocystine, la sélénite, la méthylsélénocystéine ou les nanoparticules de sélénium.

5. - Composition pour l'utilisation selon l'une quelconque des revendications 1 à 4, dans laquelle la maladie associée à l'hypertriglycéridémie, l'hypercholestérolémie et/ou l'hyperlipidémie est la maladie coronarienne (CHD), l'accident vasculaire cérébral, la maladie vasculaire périphérique (PVD), l'hypertension, l'artériosclérose, le diabète ou une combinaison de ceux-ci .

6. - Composition pour l'utilisation selon l'une quelconque des revendications 1 à 5, dans laquelle le sujet a déjà subi une thérapie par un composé de statine.

7. - Composition pour l'utilisation selon l'une quelconque des revendications 1 à 6, dans laquelle le composé de statine est l'atorvastatine (par exemple LIPITOR, TORVAST), la cérivastatine (par exemple LIPOBAY, BAYCOL ; qui ont été retirés du marché en août 2001 en raison du risque de rhabdomyolyse), la fluvastatine (par exemple LESCOL, LESCOL XL), la lovastatine (par exemple MEVACOR, ALTOCOR, ALTOPREV), la mévastatine (par exemple COMPACTIN), la pitavastatine (par exemple LIVALO, PITAVA), la pravastatine (par exemple PRAVACHOL, SELEKTINE, LIPOSTAT), la rosuvastatine (CRESTOR), la simvastatine (par exemple ZOCOR, LIPEX) ou une combinaison de celles-ci.

8. - Composition pour l'utilisation selon la revendication 6, dans laquelle le patient est initialement sous thérapie par statine avant l'administration de ladite composition, et le dosage du composé de statine est abaissé après l'administration de ladite composition.

9. - Composition pour l'utilisation selon la revendication 6, dans laquelle ladite méthode comprend l'administration de ladite composition avec un troisième médicament qui est un inhibiteur de l'absorption du cholestérol, une résine de liaison aux acides biliaires ou un fibrate.

10. - Composition pour l'utilisation selon l'une quelconque des revendications 1 à 9, dans laquelle la composition est destinée à une administration orale, nasale, topique, épidermique, buccale, vaginale ou rectale.
